# EUROPÄISCHE PATENTSCHRIFT

(11) **EP 0 548 635 B1**
(45) Veröffentlichungstag und Bekanntmachung des Hinweises auf die Patenterteilung: **06.09.1995**
(21) Anmeldenummer: 92120774.2
(22) Anmeldetag: 05.12.1992
(51) Int. Cl.: A61K 31/40, A61K 9/20, A61K 9/26, A61K 47/36

(54) **Pharmazeutische Zubereitung von Roxindol oder Carmoxirol mit vorgeqüollener Stärke**
Pharmaceutical compositions of roxindol or carmoxirol with pregelatinized starch
Compositions pharmaceutiques de roxindol ou carmoxirol utilisant de l'amidon prégelatiné

(30) Priorität: 14.12.1991 DE 4141268
(43) Veröffentlichungstag der Anmeldung: 30.06.1993
(73) Patentinhaber: MERCK PATENT GmbH, D-64271 Darmstadt (DE)
(72) Erfinder: Bolz, Joachim, Dr., W-6900 Heidelberg (DE); Batu, Atilla, W-6100 Darmstadt (DE); Seeger, Herbert, W-6101 Modautal 2 (DE)

(56) Entgegenhaltungen:
- EP-A- 0 144 012
- EP-A- 0 390 960
- EP-A- 0 451 433
- WO-A-88/01998
- 'Handbook of Pharmaceutical Excipients' 1986 , AMERICAN PHARMACEUTICALASSOCIATION , WASHINGTON, DC, USA

## Beschreibung

Gegenstand der Erfindung ist eine neue pharmazeutische Zubereitung, enthaltend als Wirkstoff eine Verbindung der Formel I 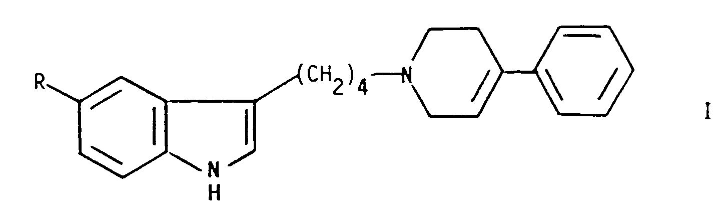
worin
- R: OH oder COOH bedeutet
oder eines ihrer pharmakologisch unbedenklichen Salze in Form einer Einbettung in vorgequollener Stärke, die mindestens einen weiteren Hilfsstoff enthalten kann.

Die Verbindungen der Formel I umschließen Roxindol (I, R = OH; vgl. EP 281 608) und Carmoxirol (I, R = COOH; vgl. EP 144 012). Sie können auch in Form ihrer pharmakologisch unbedenklichen Salze verwendet werden, insbesondere ihrer Säureadditionssalze, oder, im Falle von Carmoxirol, auch ihrer Salze mit Basen. Als Säureadditionssalze können Salze mit anorganischen Säuren verwendet werden, z.B. mit Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner Salze mit organischen Säuren, insbesondere mit aliphatischen, alicyclischen, araliphatischen, aromatischen oder heterocyclischen ein- oder mehrbasigen Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Roxindol wird vorzugsweise in Form des Methansulfonats verwendet, Carmoxirol in Form des Hydrochlorids.

Für Carmoxirol kommen als Salze mit Basen insbesondere das Natrium-, Kalium-, Magnesium-, Calcium- und Ammoniumsalz in Betracht, ferner substituierte Ammoniumsalze, z.B. das Dimethyl-, Diethyl- oder Diisopropylammonium-, Monoethanol-, Diethanol- und Triethanolammonium-, Cyclohexylammonium-, Dicyclohexylammonium- und Dibenzylethylendiammonium-Salz.

Roxindol ist ein Dopamin-Agonist und wird als Neuroleptikum verwendet. Carmoxirol ist ein dopaminerger Blutdrucksenker.

Bei Versuchen, haltbare und in reproduzierbarer Weise stabile pharmazeutische Zubereitungen, insbesondere für die orale Applikation, von Roxindol und Carmoxirol herzustellen, ergaben sich jedoch erhebliche Schwierigkeiten. So gelang es mit einer ganzen Reihe üblicher Methoden nicht, dieses Ziel zu erreichen: Herstellung von Granulaten, z.B. mit Lactose-hydrat, Mannit oder mikrokristalliner Cellulose als Füllstoff, insbesondere auch unter Verwendung von Lösungen von Celluloseethern oder Gelatine; Einbettungen in Gelatine mit anschließender Zugabe von Füllstoffen; Aufziehen der Wirkstoffe in Lösung, z.B. auf Lactose-hydrat, Mannit oder mikrokristalline Cellulose; Wirkstoffverreibung mit Lactose-hydrat oder vorgequollener Maisstärke.

Auch Stabilisierungsversuche der genannten Zubereitungen mit Antioxydantien wie Ascorbinsäure, Tocopherol oder Tocopherol-derivaten oder mit Citronensäure gaben nicht den gewünschten Erfolg.

Der Erfindung lag die Aufgabe zugrunde, eine stabile pharmazeutische Zubereitung für die genannten Wirkstoffe aufzufinden.

Es wurde überraschend gefunden, daß Einbettungen dieser Wirkstoffe in vorgequollener Stärke im Gegensatz zu den anderen genannten Zubereitungen die notwendigen Anforderungen an Haltbarkeit und Stabilität erfüllen.

Vorgequollene Stärke ("pregelatinized starch"), insbesondere vorgequollene Maisstärke, aber auch Kartoffel-, Weizen-, Reis- oder Tapiokastärke ist bekannt und im Handel erhältlich. Aufgrund ihrer Neigung zum Quellen wird sie in der Regel in ähnlichen Fällen nicht als Wirkstoffträger eingesetzt. Ihre Verwendung zur Herstellung der erfindungsgemäßen Zubereitungen bedeutet daher die Überwindung eines Vorurteils der Fachwelt.

Ein bevorzugtes Handelsprodukt ist eine vorgequollene Maisstärke mit folgenden Kenndaten:
Korngröße: mindestens 90 % < 150 µ
Schüttvolumen: 1,6 ml/g
Stampfvolumen: 1,3 ml/g
Kalt-wasserlösliche Anteile: 10-20 %
Trocknungsverlust: maximal 14 %
pH-Wert einer Dispersion in Wasser: 4,5-7.

Die erfindungsgemäße Einbettung kann einen oder mehrere weitere(n) Hilfsstoff(e) enthalten. Als solche kommen z.B. in Frage: Gleitmittel wie Magnesium- oder Calciumstearat, Calciumbehenat, Talk; Bindemittel wie Cellulose, insbesondere mikrokristalline Cellulose; Fließregulierungsmittel wie hochdisperses Siliciumdioxid; Sprengmittel wie die Natriumsalze von Carboxymethylcellulose oder Carboxymethylstärke oder Polyvinylpyrrolidon; Füllstoffe wie Lactose oder Dextrine.

Die Einbettung enthält vorzugsweise 0,1 bis 5, insbesondere 0,5 bis 2 (Gewichtsprozent) Wirkstoff, 70 bis 99,9, insbesondere 80 bis 99 vorgequollene Stärke und 0 bis 29,9, insbesondere 0,3 bis 20 weitere(n) Hilfsstoff(e).

Die Einbettung kann wie nachstehend angegeben hergestellt und in übliche Darreichungsformen für die orale Applikation, insbesondere Tabletten oder Kapseln, aber auch Pellets, übergeführt werden.

Gegenstand der Erfindung ist demnach ferner ein neues Verfahren zur Herstellung einer pharmazeutischen Zubereitung nach Anspruch 1, dadurch gekennzeichnet, daß man (a) eine Lösung des Wirkstoffs auf vorgequollene Stärke (oder auf ein Gemisch aus vorgequollener Stärke und mindestens einem weiteren Hilfsstoff) aufzieht, oder (b) den Wirkstoff in mikronisierter Form mit vorgequollener Stärke (oder mit einem Gemisch aus vorgequollener Stärke und mindestens einem weiteren Hilfsstoff) mischt sowie Wasser oder ein Wasser-Alkohol-Gemisch zugibt, das nach (a) oder (b) erhaltene Gemisch, falls erforderlich, knetet, sodann siebt und trocknet und gegebenenfalls die so erhaltene Einbettung in üblicher Weise in Tabletten oder Kapseln überführt.

Der Wirkstoff wird vorzugsweise in Ethanol oder in einem Gemisch aus Ethanol und Wasser oder aus Aceton und Wasser gelöst.

Es ist vorteilhaft, wenn man alle Verfahrensschritte bei Temperaturen zwischen 10 und 40°, vorzugsweise zwischen 20 und 30° durchführt.

Erhaltene Tabletten können, falls erwünscht, in üblicher Weise lackiert oder mit einem - gegebenenfalls magensaftresistenten oder auf andere Weise retardierenden - Tablettenüberzug versehen werden.

Die erfindungsgemaße Zubereitung ist stabil insbesondere gegen oxydative Zersetzung und ermöglicht eine schnelle Freisetzung des Wirkstoffes (wenn man nicht auf eine Retardierung Wert legt).

### Beispiel 1: Hartgelatine-Kapsel 1 mg

Man löst 200 g Roxindol-methansulfonat bei 30° in einem Gemisch von 3 l Ethanol und 3 l Wasser. In einem Kneter werden 27 725 g vorgequollene Maisstärke vorgelegt, die erhaltene Lösung wird auf die Maisstärke aufgetragen. Die Masse wird geknetet und durch ein Granuliersieb (1 mm lichte Weite) gegeben. Man trocknet das so erhaltene Granulat unter vermindertem Druck bis zu einem Wassergehalt von 5-9 %, siebt erneut (0,8 mm lichte Weite), vermischt mit 75 g Magnesiumstearat und füllt das so erhaltene Produkt in Hartgelatinekapseln (Größe 4; Leergewicht einer Kapsel 40 mg). Jede Kapsel enthält 1 mg Roxindol-methansulfonat, 138,625 mg vorgequollene Stärke und 0,375 mg Magnesiumstearat.

Analog erhält man Kapseln, die an Stelle des Roxindol-methansulfonats als Wirkstoff jeweils 1 mg Carmoxirol-hydrochlorid enthalten.

### Beispiel 2: Lacktabletten 2,5 mg

Man löst 50 g Roxindol-methansulfonat bei 30° in einem Gemisch von 300 ml Ethanol und 300 ml Wasser. In einem Kneter werden 2330 g vorgequollene Maisstärke und 400 g mikrokristalline Cellulose vorgelegt, die erhaltene Lösung wird aufgetragen. Man arbeitet weiter analog Beispiel 1, vermischt sodann mit 20 g Magnesiumstearat und preßt das Gemisch zu Tablettenkernen (7 mm Durchmesser, 6 mm Wölbungsradius), die jeweils aus 2,5 mg Roxindol, 116,5 mg vorgequollener Stärke, 20 mg mikrokristalliner Cellulose und 1 mg Magnesiumstearat bestehen. Die Kerne werden anschließend in üblicher Weise in einem Coater lackiert, wobei man eine Suspension bestehend aus 14 g Titandioxid, 20 g gelbem Eisenoxid, 0,04 g rotem Eisenoxid, 36 g Hydroxypropylmethylcellulose, 9 g Polyethylenglykol 400, 300 ml Ethanol und 300 ml Wasser verwendet. Die erhaltenen Lacktabletten werden bei 30° getrocknet.

Analog erhält man Lacktabletten, die an Stelle des Roxindol-methansulfonats als Wirkstoff jeweils 2,5 mg Carmoxirol-hydrochlorid enthalten.

### Beispiel 3: Hartgelatinekapseln 2 mg

Man mischt 40 g Carmoxirol-hydrochlorid mit zunächst 500 g, dann weiteren 2245 g vorgequollener Maisstärke, gibt ein Gemisch von 400 ml Ethanol und 400 ml Wasser hinzu und knetet 10 Minuten. Die feuchte Masse wird durch ein Granuliersieb (1 mm lichte Weite) gegeben. Man trocknet das so erhaltene Granulat unter vermindertem Druck bis zu einem Wassergehalt von 5-9 %, siebt erneut (0,8 mm lichte Weite), vermischt mit 5 g hochdispersem Siliciumdioxid und 10 g Magnesiumstearat und füllt das so erhaltene Produkt in Hartgelatinekapseln (Größe 4). Jede Kapsel enthält 2 mg Carmoxirolhydrochlorid, 137,25 mg vorgequollene Stärke, 2,5 mg Siliciumdioxid und 5 mg Magnesiumstearat.

Analog erhält man Kapseln, die an Stelle des Carmoxirol-hydrochlorids als Wirkstoff jeweils 2 mg Roxindol-methansulfonat enthalten.

### Beispiel 4: Lacktabletten 2 mg

Man mischt 40 g mikronisiertes Carmoxirol-hydrochlorid mit 500 g vorgequollener Kartoffelstärke, gibt sodann weitere 1445 g vorgequollene Kartoffelstärke und 400 g mikrokristalline Cellulose hinzu und mischt erneut. Das Gemisch wird mit einer Mischung von 400 ml Ethanol und 400 ml Wasser versetzt und 10 Minuten durchgeknetet. Man gibt durch ein Granuliersieb (1 mm lichte Weite), trocknet unter vermindertem Druck bis zu einem Wassergehalt von 5-9 %, siebt erneut (0,8 mm lichte Weite), vermischt mit 5 g hochdispersem Siliciumdioxid und 10 g Magnesiumstearat und preßt das so erhaltene Gemisch zu Tablettenkernen (7 mm Durchmesser, 6 mm Wölbungsradius), die jeweils aus 2 mg Carmoxirol-hydrochlorid, 97,25 mg vorgequollener Kartoffelstärke, 20 mg mikrokristalliner Cellulose, 0,25 mg hochdispersem Siliciumdioxid und 0,5 mg Magnesiumstearat bestehen. Die Kerne werden anschließend analog Beispiel 2 lackiert.

### Beispiel 5: Lacktabletten 5 mg

Analog Beispiel 2 erhält man Lacktabletten, deren Kerne folgende Zusammensetzung haben: 5 mg Roxindol-methansulfonat, 200 mg vorgequollene Reisstärke, 73 mg mikrokristalline Cellulose, 2 mg Magnesiumstearat.

## Patentansprüche (Patentansprüche für folgende(n) Vertragsstaat(en): AT, BE, CH, DE, DK, FR, GB, GR, IE, IT, LI, LU, NL, PT, SE)

1. Pharmazeutische Zubereitung, enthaltend als Wirkstoff eine Verbindung der Formel I 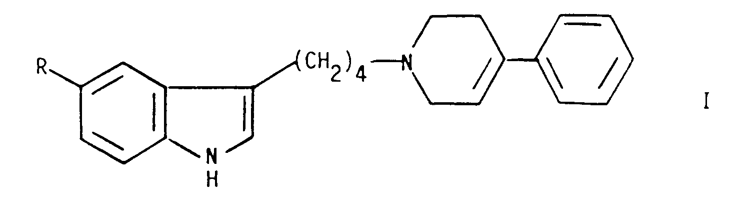 worin
R OH oder COOH bedeutet
oder eines ihrer pharmakologisch unbedenklichen Salze in Form einer Einbettung in vorgequollener Stärke, die mindestens einen weiteren Hilfsstoff enthalten kann.

2. Pharmazeutische Zubereitung nach Anspruch 1 in Form einer Tablette oder einer Kapsel.

3. Verfahren zur Herstellung einer pharmazeutischen Zubereitung nach Anspruch 1, dadurch gekennzeichnet, daß man (a) eine Lösung des Wirkstoffs auf vorgequollene Stärke (oder auf ein Gemisch aus vorgequollener Stärke und mindestens einem weiteren Hilfsstoff) aufzieht, oder (b) den Wirkstoff in mikronisierter Form mit vorgequollener Stärke (oder mit einem Gemisch aus vorgequollener Stärke und mindestens einem weiteren Hilfsstoff) mischt sowie Wasser oder ein Wasser-Alkohol-Gemisch zugibt, das nach (a) oder (b) erhaltene Gemisch, falls erforderlich, knetet, sodann siebt und trocknet und gegebenenfalls die so erhaltene Einbettung in üblicher Weise in Tabletten oder Kapseln überführt.

## Patentansprüche (Patentansprüche für folgende(n) Vertragsstaat(en): ES)

1. Verfahren zur Herstellung einer pharmazeutischen Zubereitung, enthaltend als Wirkstoff eine Verbindung der Formel I 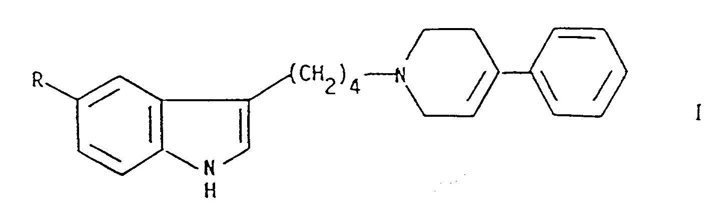 worin
R OH oder COOH bedeutet
oder eines ihrer pharmakologisch unbedenklichen Salze in Form einer Einbettung in vorgequollener Stärke, die mindestens einen weiteren Hilfsstoff enthalten kann.

2. Verfahren zur Herstellung einer pharmazeutischen Zubereitung nach Anspruch 1 in Form einer Tablette oder Kapsel.

3. Verfahren zur Herstellung einer pharmazeutischen Zubereitung nach Anspruch 1, dadurch gekennzeichnet, daß man (a) eine Lösung des Wirkstoffs auf vorgequollene Stärke (oder auf ein Gemisch aus vorgequollener Stärke und mindestens einem weiteren Hilfsstoff) aufzieht oder (b) den Wirkstoff in mikronisierter Form mit vorgequollener Stärke (oder mit einem Gemisch aus vorgequollener Stärke und mindestens einem weiteren Hilfsstoff) mischt, sowie Wasser oder ein Wasser-Alkohol-Gemisch zugibt, das nach (a) oder (b) erhaltene Gemisch, falls erforderlich, knetet, sodann siebt und trocknet und gegebenenfalls die so erhaltene Einbettung in üblicher Weise in Tabletten oder Kapseln überführt.

## Claims (Claims for the following Contracting State(s): AT, BE, CH, DE, DK, FR, GB, GR, IE, IT, LI, LU, NL, PT, SE)

1. Pharmaceutical formulation containing as the active ingredient a compound of formula I: 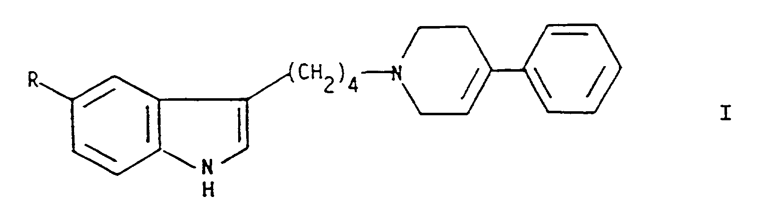 wherein
R is OH or COOH,
or one of its pharmacologically acceptable salts, in the form of an inclusion in pregelatinised starch, which can contain at least one other adjunct.

2. Pharmaceutical formulation according to Claim 1, in the form of a tablet or a capsule.

3. Process for the preparation of a pharmaceutical formulation according to Claim 1, characterized in that
(a) a solution of the active ingredient is absorbed on to pregelatinised starch (or on to a mixture of pregelatinised starch and at least one other adjunct), or
(b) the active ingredient in micronised form is mixed with pregelatinised starch (or with a mixture of pregelatinised starch and at least one other adjunct) and water or a water/alcohol mixture is added, the mixture obtained according to (a) or (b) is kneaded, if necessary, and then sieved and dried and, if appropriate, the resulting inclusion is converted to tablets or capsules in conventional manner.

## Claims (Claims for the following Contracting State(s): ES)

1. Process for the preparation of a pharmaceutical formulation containing as the active ingredient a compound of formula I: 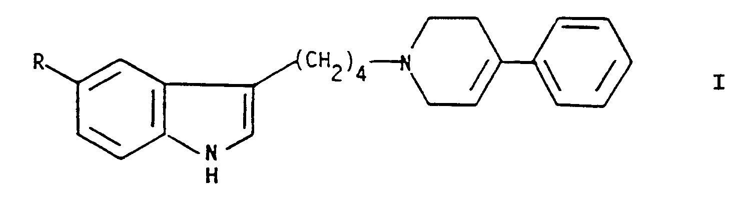 wherein
R is OH or COOH,
or one of its pharmacologically acceptable salts, in the form of an inclusion in pregelatinised starch, which can contain at least one other adjunct.

2. Process for the preparation of a pharmaceutical formulation according to Claim 1, in the form of a tablet or capsule.

3. Process for the preparation of a pharmaceutical formulation according to Claim 1, characterized in that
(a) a solution of the active ingredient is absorbed on to pregelatinised starch (or on to a mixture of pregelatinised starch and at least one other adjunct), or
(b) the active ingredient in micronised form is mixed with pregelatinised starch (or with a mixture of pregelatinised starch and at least one other adjunct) and water or a water/alcohol mixture is added, the mixture obtained according to (a) or (b) is kneaded, if necessary, and then sieved and dried and, if appropriate, the resulting inclusion is converted to tablets or capsules in conventional manner.

## Revendications (Revendications pour l'(les) Etat(s) contractant(s) suivant(s): AT, BE, CH, DE, DK, FR, GB, GR, IE, IT, LI, LU, NL, PT, SE)

1. Composition pharmaceutique contenant en tant que substance active un composé de formule I 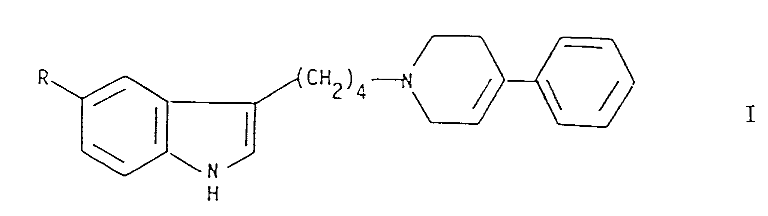 dans laquelle
R représente OH ou COOH,
ou l'un de ses sels acceptables pour l'usage pharmaceutique, à l'état enrobé dans de l'amidon prégélatinisé, la composition pouvant contenir au moins un autre produit auxiliaire.

2. Composition pharmaceutique selon revendication 1, à l'état de comprimés ou de capsules.

3. Procédé pour préparer une composition pharmaceutique selon revendication 1, caractérisé en ce que (a) on absorbe une solution de la substance active sur l'amidon prégélatinisé (ou sur un mélange d'amidon prégélatinisé et d'au moins un produit auxiliaire), ou bien (b) on mélange la substance active à l'état micronisé avec de l'amidon prégélatinisé (ou avec un mélange d'amidon prégélatinisé et d'au moins un autre produit auxiliaire) et on ajoute de l'eau ou un mélange eau-alcool, on malaxe lorsque c'est nécessaire le mélange obtenu en (a) ou (b) puis on tamise et on sèche et le cas échéant, on met la composition enrobée ainsi obtenue, de la manière habituelle, à l'état de comprimés ou de capsules.

## Revendications (Revendications pour l'(les) Etat(s) contractant(s) suivant(s): ES)

1. Procédé de préparation d'une composition pharmaceutique contenant en tant que substance active un composé de formule I 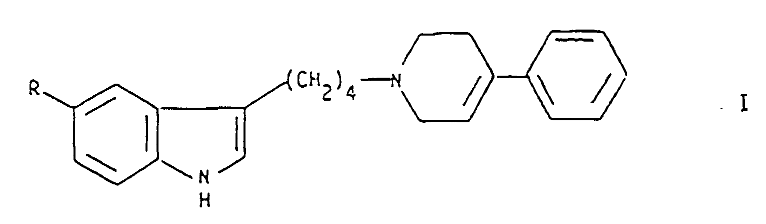 dans laquelle
R représente OH ou COOH,
ou l'un de ses sels acceptables pour l'usage pharmaceutique, à l'état enrobé dans de l'amidon prégélatinisé, la composition pouvant contenir au moins un autre produit auxiliaire.

2. Procédé de préparation d'une composition pharmaceutique selon la revendication 1, à l'état de comprimés ou de capsules.

3. Procédé de préparation d'une composition pharmaceutique selon la revendication 1, caractérisé en ce que (a) on absorbe une solution de la substance active sur l'amidon prégélatinisé (ou sur un mélange d'amidon prégélatinisé et d'au moins un produit auxiliaire), ou bien (b) on mélange la substance active à l'état micronisé avec de l'amidon prégélatinisé (ou avec un mélange d'amidon prégélatinisé et d'au moins un autre produit auxiliaire) et on ajoute de l'eau ou un mélange eau-alcool, on malaxe lorsque c'est nécessaire le mélange obtenu en (a) ou (b) puis on tamise et on sèche et le cas échéant, on met la composition enrobée ainsi obtenue, de la manière habituelle, à l'état de comprimés ou de capsules.
